# EUROPEAN PATENT APPLICATION

(11) **EP 1 486 563 A1**
(43) Date of publication of application: **15.12.2004**
(21) Application number: 03707149.5
(22) Date of filing: 27.02.2003
(51) Int. Cl.: C12N 15/09, C12N 5/10, A01K 67/027

(54) **EMBRYONICALLY MODIFIED ANIMAL AND METHOD OF CONSTRUCTING THE SAME**

(30) Priority: 27.02.2002 JP 2002051302
(71) Applicant: Nagao, Yasumitsu, Kyoto-shi, Kyoto 606-8237 (JP); Imai, Hiroshi, Otsu-shi, Shiga 520-0014 (JP); Horii, Takuro, Maebashi-shi, Gunma 371-0031 (JP); Totsuka, Yoshikazu, Utsunomiya-shi, Tochigi 320-0065 (JP)
(72) Inventor: Nagao, Yasumitsu, Kyoto-shi, Kyoto 606-8237 (JP); Imai, Hiroshi, Otsu-shi, Shiga 520-0014 (JP); Horii, Takuro, Maebashi-shi, Gunma 371-0031 (JP); Totsuka, Yoshikazu, Utsunomiya-shi, Tochigi 320-0065 (JP)
(74) Representative: Denison, Christopher Marcus
(86) International application number: PCT/JP2003/002265
(87) International publication number: WO 2003/072777

(57) **Abstract**

The present invention relates to a method for efficiently producing a reproducible animal using totipotent cells wherein mitochondrial DNAs (e.g., wild-type DNAs) adapted to nuclear DNAs have been introduced into or substituted with mitochondrial DNAs, and the present invention also relates to an animal obtained by such production method. When the totipotent cells are ES cells derived from an inbred mouse, the tetraploid rescue method is preferably used. In the production of chimeric animals, mitochondrial DNAs of totipotent cells derived from an animal to be used is substituted with wild-type mitochondrial DNAs by the back-crossing method, the nuclear replacement method, or the like, and the cells are injected into a tetraploid fertilized egg, so that a reproducible inbred chimeric animal is produced while avoiding death of the obtained inbred chimeric animal from respiratory disturbances and the like immediately after birth. The thus obtained reproducible chimeric animal can be used for gene function analyses, animal experiments, and the like without carrying out complicated manipulations for generating inbred animals.

SEQUENCE LISTING

<110> Nagao, Yasumitsu; Imai, Hiroshi; Horii, Takuro
      Totsuka, Yoshikazu

<120> Animal Derived from Manipulated Embryo and Preparing Method thereof

<130> PH-2203-PCT

<150> JP 2002-51302
<151> 2002-2-27

<160> 2

<210> 1
<211> 17
<212> DNA
<213> Artificial Sequence

<400> 1
aattattttc cccaagc                                                17

<210> 2
<211> 15
<212> DNA
<213> Artificial Sequence

<400> 2
agaagagggg cattg                                                  15

## Description

### [TECHNICAL FIELD]

The present invention relates to a method for efficiently producing a reproducible animal derived from manipulated embryo that shows germ-line transmission when gene-altered animals such as knockout mice are produced. More specifically, when totipotent cells such as embryonic stem cells (ES cells) are produced, the present invention relates to a method for producing an animal derived from totipotent cells that is prepared by back-crossing, cell fusion, mitochondria injection, pronuclei replacement, somatic nuclear transfer, or the like to introduce or substitute a desired type of mitochondrial DNAs. Particularly when an animal species is a mouse, the present invention relates to a method for efficiently producing reproducible inbred germ-line chimeric mice by improving the birth rate and the survival rate of newborns.

The present invention further relates to a reproducible mouse that is produced by any one of the above methods, wherein mitochondrial DNAs are adapted type mitochondrial DNAs that are of a genotype by which no nuclear-mitochondrial incompatibility takes place, and the totipotent cells are transmitted to the germ line.

### [BACKGROUND ART]

Conventionally, technology exists for producing chimeric mice preceding the technology for production of so-called gene-targeted mice such as knockout mice. Chimeric mice are produced using ES cells that have been proven to be transmitted to a germ line. As such ES cells proven to be transmitted to a germ line, TT2 cells of an ES cell line established from hybrids of C57BL/6 mice and CBA mice and ES cell lines established from mice 129 substrains, which are non-standard strains (there are 3 standard strains of mouse: BALB/c, C57BL/6, and C3H), are used. Hence, to use chimeric mice in phenotype analyses or as experimental animals in gene function analyses and the like, there is a need to introduce target gene alteration in the produced mice (gene-altered mice) into inbred mice by a back-crossing method. Specifically, complicated procedures that have been required include producing gene-altered ES cells using ES cells of a hybrid strain or non-standard strains, producing germ-line chimeric mice using the cells, and then repeatedly back-crossing the thus obtained hetero-type gene-altered mice (generally, male mice are used) with inbred mice of a standard strain (e.g., C57BL/6), so as to generate inbred mice of the standard strain.

In contrast, direct production of germ-line chimeric mice of an inbred strain has been attempted by establishing ES cells from inbred mice. However, although there have been reports that germ-line chimeric mice could be obtained from ES cells derived from inbred mice such as C57BL/6 above, this has not yet been practically applied. Currently, hybrid strains are used as described above. Therefore, it is substantially difficult to obtain ES cells of good inbred mice to obtain germ-line chimeric mice.

In addition, it is said that ontogenesis using only ES cells is substantially impossible.

In the meantime, a tetraploid rescue method has been devised as a method for selectively producing chimeric mice from ES cells (Nagy A et al., Development 110, 815-821 (1990)). This method is based on the knowledge that tetraploid cells develop into placenta, and only ES cells develop into an individual body when they are injected into tetraploid fertilized eggs. This technique is called the tetraploid rescue method. However, according to a recent report concerning comparison and examination conducted for the tetraploid rescue method and nuclear transfer technology, most newborns died because of respiratory disturbances after birth and only one inbred chimeric mouse could be produced and obtained by the tetraploid rescue method, when ES cells established from BALB/c mice were used (Eggan K et al., PNAS 98, 6209-6214 (2001)). Furthermore, also regarding clone mouse production using somatic nuclear transfer technology, it has been reported that most newborns of the obtained inbred mice died after birth because of respiratory disturbances, and viable newborn clone mice could be obtained through the use of cell nuclei of hybrid mice (Wakayama T et al., Nature 394, 369-374 (1998); Wakayama T et al., PNAS 96, 1484-1498 (1999); Humpherys D et al., Science 296, 95-97 (2001)). As described above, even if no apparent abnormalities are observed, chimeric mice generated by the tetraploid rescue method only from ES cells of inbred mice and clone mice derived from somatic cell nuclei of inbred mice die in many cases because of respiratory disturbances. Such problem is inferred to take place when an animal species is changed to mammals other than mice.

Moreover, successfully produced conventional knockout mice are germ-line chimeras obtained using ES cells derived from mice of a hybrid strain or mice 129 substrains, non-standard strains, but are not inbred mice. Reports have been extremely rare that when ES cells derived from inbred mice were used, chimeric mice could be produced. None has been reported subsequently. No germ-line transmission has been reported in most successful cases, and it has been substantially impossible to obtain germ-line chimeric mice. As described above, the tetraploid rescue method has been thought to be the sole means to address the problems accompanying inbred chimeric mouse production. However, most newborns obtained by this method die immediately after birth because of respiratory disturbances (Eggan K et al., PNAS 98, 6209-6214 (2001)). Thus, it has been impossible to actually obtain reproducible chimeric mice.

### [SUMMARY OF THE INVENTION]

Under the above circumstances, we have intensively studied the production of animals derived from manipulated embryo such as reproducible chimeric animals. In particular, we have studied the production of a chimeric mouse, wherein mitochondrial DNAs are adapted type (the mitochondrial DNAs, by which no nuclear-mitochondrial incompatibility, such as wild-type mitochondrial DNAs, takes place) and totipotent cells are transmitted to a germ line.

Specifically, an object of the present invention is to efficiently obtain animals derived from manipulated embryos such as reproducible chimeric animals, in particular, inbred chimeric animals, and further particularly, inbred chimeric mouse individuals. Particularly, an object of the present invention is to develop a method for producing: inbred chimeric animals derived from totipotent cells such as ES cells derived from inbred animals used in production of chimeric animals obtained by the tetraploid rescue method; and inbred chimeric animals and particularly inbred chimeric mice, while avoiding death of the inbred chimeric mice immediately after birth because of respiratory disturbances.

Specifically, the object of the present invention is, in production of chimeric animals by the tetraploid rescue method, to provide a method for efficiently producing: inbred chimeric animals derived from totipotent cells such as ES cells derived from inbred animals used; and reproducible inbred chimeric animals and particularly inbred chimeric mice, while particularly avoiding death of inbred chimeric mice immediately after birth because of respiratory disturbances.

A second object of the present invention is to provide reproducible inbred chimeric mice that are animals derived from manipulated embryo, such as chimeric animals obtained by the above production method, whose rate of death immediately after birth because of respiratory disturbances is particularly low, and wherein mitochondrial DNAs are adapted type mitochondrial DNAs and totipotent cells are transmitted to a germ line.

Furthermore, a third object of the present invention relates to totipotent cells and particularly embryonic stem cells that are used for producing such animal derived from a manipulated embryo.

Totipotent cells in the present invention mean undifferentiated cells capable of differentiating into any type of cell. Examples of such cells include embryonic stem cells and nuclear transferred embryos.

Characteristics of the present invention relate to: a method for producing the above-described reproducible animals derived from manipulated embryo and particularly reproducible germ-line chimeric animals; reproducible chimeric animals, and particularly chimeric mice and reproducible inbred chimeric mice produced by such method; and totipotent cells used for the production.

Specifically, the present invention is a method that can be applied for animals derived from manipulated embryos, and particularly chimeric mice obtained as a result of production of gene-altered mice such as so-called knockout mice. The method is characterized in that it involves introducing adapted type mitochondrial DNAs into a cell to be subjected to injection or substituting mitochondrial DNAs of the cell to be subjected to injection with adapted type mitochondrial DNAs, or using a cell wherein mitochondrial DNAs of a host embryo has been introduced therein or substituted with adapted type mitochondrial DNAs. In this case, totipotent cells having adapted type mitochondrial DNAs introduced therein and an untreated host embryo, untreated totipotent cells and a host embryo having adapted type mitochondrial DNAs introduced therein, totipotent cells having adapted type mitochondrial DNAs introduced therein and a host embryo having adapted type mitochondrial DNAs introduced therein, or the like can be used. The thus produced chimeric animal has the adapted type mitochondrial DNAs. Examples of such adapted type mitochondrial DNAs include wild-type-derived mitochondrial DNAs, and a Mus musculus musculus-type DNAs of a wild-type mouse. When germ-line chimeric animals derived from totipotent cells such as ES cells produced from inbred animals and particularly chimeric mice completely derived from ES cells are produced, totipotent cells such as ES cells of an inbred mouse wherein mitochondrial DNAs have been introduced or substituted is used as cells to be injected into a tetraploid fertilized egg. In this manner, death of the obtained inbred chimeric mice immediately after birth because of respiratory disturbances and the like can be avoided, and reproducible inbred chimeric mice can be efficiently produced.

To date, when inbred chimeric mice selectively derived from ES cells are produced by the tetraploid rescue method using the ES cells of inbred mice, most of the obtained chimeric mice died immediately after birth because of respiratory disturbances. It has actually been impossible to obtain reproducible chimeric mice by the tetraploid rescue method. The existence of abnormal DNA methylation patterns has been posed as a reason why most newborns obtained by the tetraploid rescue method die because of respiratory disturbances (Dean W et al., Development 125, 2237-2282(1998)). In addition to this reason, we were able to confirm for the first time that incompatibility between mitochondrial DNAs and nuclear DNAs is also a reason. Hence, we could have avoided death immediately after birth by converting mitochondrial DNAs of ES cells derived from an inbred mouse into mitochondrial DNAs of a wild-type mouse for the DNAs able to be adapted to nuclear DNAs. When we applied this technology to inbred animals (mice), we could efficiently produce germ-line chimeric mice. We believe that we can expect a similar effect also in the case of clone animals and general diploid chimeric animals if adapted type mitochondrial DNAs are introduced into animals that are produced by embryo manipulation.

The present invention provides the following methods, an animal derived from a manipulated embryo and particularly a chimeric animal produced by such methods, and totipotent cells.
1. A method for producing an animal derived from manipulated embryo derived from totipotent cells, comprising using cells having adapted type mitochondrial DNAs introduced therein which adapt to nuclear DNAs.
2. The method for producing an animal of 1 above, wherein the animal derived from the manipulated embryo derived from the totipotent cells is a chimeric animal.
3. The method for producing an animal of 2 above, wherein the chimeric animal is an inbred chimeric animal.
4. The method for producing an animal of any one of 1 to 3 above, comprising using totipotent cells having adapted type mitochondrial DNAs introduced therein and an untreated host embryo, untreated totipotent cells and a host embryo having adapted type mitochondrial DNAs introduced therein, or totipotent cells having adapted type mitochondrial DNAs introduced therein and a host embryo having adapted type mitochondrial DNAs introduced therein.
5. The method for producing an animal of 4 above, comprising using a tetraploid fertilized egg as a host embryo for an animal and totipotent cells as cells having adapted type mitochondrial DNAs introduced therein.
6. The method for producing an animal of 5 above according to a tetraploid rescue method, comprising injecting the totipotent cells having the adapted type mitochondrial DNAs introduced therein into the blastocyst of a tetraploid fertilized egg.
7. The method for producing an animal of any one of 1 to 6 above, wherein the cells having the adapted type mitochondrial DNAs introduced therein are cells which have the adapted type mitochondrial DNAs as a result of substitution.
8. A method for producing a clone individual by nuclear transfer using a recipient oocyte having adapted type mitochondrial DNAs that adapt to nuclear DNAs of a donor cell.
9. The method for producing an animal of any one of 1 to 8 above, wherein nuclear-mitochondrial incompatibility results in respiratory disturbances immediately after birth.
10. The method for producing an animal of any one of 1 to 9 above, wherein the adapted type mitochondrial DNAs are mitochondrial DNAs derived from a wild type.
11. The production method of any one of 1 to 10 above, wherein the animal is a mouse.
12. The method for producing an animal of any one of 1 to 11 above, wherein the method for substituting mitochondrial DNAs with desired mitochondrial DNAs is either a back-crossing method or a pronuclei replacement method.
13. The method for producing an animal of any one of 9 to 12 above, wherein the desired mitochondrial DNAs are of a wild-type mouse Mus musculus musculus type.
14. A reproducible chimeric animal, which is produced by any one of the methods of 1 to 13 above, and wherein mitochondrial DNAs areintroduced into or substituted with adapted type DNAs, and totipotent cells can be transmitted to a germ line.
15. The animal of 14 above, wherein the animal is a mouse.
16. A totipotent cell for producing a reproducible genetically manipulated animal, wherein mitochondrial DNAs have been introduced into or substituted with adapted type DNAs, and its character is transmitted to a germ line.
17. The totipotent cell of 16 above, wherein adaptation with nuclear DNAs cause a decrease in respiratory disturbances immediately after birth.
18. The totipotent cell of 16 or 17 above, wherein adapted type mitochondrial DNAs have been introduced as mitochondrial DNAs.
19. The totipotent cell of 18 above, wherein the adapted type mitochondrial DNAs areof a wild-type mouse Mus musculus musculus type.
20. The embryonic stem cell of any one of 16 to 19 above, wherein the genetically manipulated animal is a mouse.

### [BEST MODE OF CARRYING OUT THE INVENTION]

It is preferable to obtain an animal derived from a manipulated embryo obtained by the method of the present invention through the use of a tetraploid rescue method using totipotent cells such as ES cells or the nuclear transfer embryo of an inbred animal. Hence, the animals obtained according to the present invention are basically inbred animals wherein most cells including germ-line cells are derived from totipotent cells of inbred animals used. Furthermore, since the mitochondrial DNAs of totipotent cells such as ES cells of an inbred animal to be used have been previously substituted with those of an animal of a wild-type species by pronuclei replacement or the like, the obtained chimeric animal grows without dying from respiratory disturbances immediately after birth. Therefore, reproducible chimeric animals can be efficiently obtained, and inbred animals that can be utilized for gene function analyses or as experimental animals can be directly obtained from the obtained germ-line chimeric animals. Hence, this is very useful because there is no need to repeat time-consuming and complicated crossing to generate inbred animals. Such animals may be any animals as long as they are animals regarding which it has been reported that chimeras can be generally produced therewith. Inbred animals are particularly preferable. Such animals may preferably be mammals, further preferably rodents, and particularly preferably mice.

Totipotent cells such as ES cells derived from these animals are prepared by a known method, and then used in the present invention. Embodiments of the use of ES cells derived from a mouse are explained as follows. It is naturally understood that the animal species used in the present invention are not particularly limited to a mouse.

A mouse-derived mitochondrial DNAs-substituted ES cells are prepared by intracellular substitution of mitochondrial DNAs according to the above conventional technology. Substitution of mitochondrial DNAs of an inbred mouse is conducted by producing an inbred mouse (in many cases, the inbred mouse is of a standard strain such as C57BL/6, C3H, or BALB/c) having mitochondrial DNAs of a desired type as a result of substitution by the back-crossing method, the nuclear transfer method, or the like, and then establishing ES cells from the mouse, so as to be able to obtain ES cells having the substituted mitochondrial DNAs to be used in the present invention. When C57BL/6 inbred mice are repeatedly crossed with Mus musculus musculus wild-type mice by the back-crossing method 12 times or more, B6-mtMus inbred mice wherein the mitochondrial DNAs of the C57BL/6 inbred mice have been substituted with those of Mus musculus musculus type of the wild-type mouse can be produced. From the mice, ES cells wherein the mitochondrial DNAs have been substituted with the Mus musculus musculus-type DNAs of an adapted type mouse can be established.

In the meantime, when the pronuclei replacement method is employed, according to the standard method (McGrath J & Solter D, J. Exp. Zool. 228, 355-362 (1983)), male and female pronuclei of the pronuclear-stage fertilized egg of an mdx inbred mouse are transplanted into an enucleated pronuclear-stage fertilized egg having a Mus musculus musculus-type mitochondria of the wild-type mouse. Nuclear fusion is carried out by electric pulses, the egg is cultured, and then the egg is transplanted into a mouse oviduct for ontogenesis, so that an mdx-mtMus inbred mouse wherein the mitochondrial DNAs have been substituted with those of the Mus musculus musculus type can be produced. Similarly, ES cells wherein the mitochondrial DNAs have been substituted with those of a wild type can be established from this mouse.

To establish ES cells, a blastocyst-stage embryo is collected from the inbred mouse produced in this manner, such as B6-mtMus or mdx-mtMus, and then ES cells can be established by a standard method (Evans MJ and Kaufman MK, Nature 292, 154-156 (1981)).

ES cells having a target character obtained by the above method is injected into an embryo of a fertilized egg, preferably a tetraploid embryo that has divided into a blastocyst-stage tetraploid fertilized egg, thereby producing a chimeric mouse.

Blastocyst-stage tetraploid embryos can be obtained by naturally mating ICR mice that had been subjected to superovulation induction treatment with male mice of the same strain, collecting late 2-cell stage embryos by flushing the oviduct method from mouse individuals for which plugs have been confirmed, conducting fusion treatment by the electric pulse method to prepare tetraploid embryos, and then culturing the embryos in M16 media or CZB media. ES cells prepared from the prescribed B6-mtMus or mdx-mtMus inbred mice are injected into the obtained blastocyst-stage embryos using a microinjector such as a piezomicromanipulator. Next, the thus obtained blastocyst-stage embryos having had the ES cells injected therein are transplanted into the uteri of recipient ICR mice after crossing and 2.5 days after plug confirmation. On the night before delivery (night of day 18.5 of pregnancy), chimeric mouse fetuses are taken out by Cesarean section, and then resuscitated. After the confirmation of active movement of the fetuses, they are raised by previously prepared foster parents. Most of the thus obtained newborns (chimeric mice) conducted spontaneous respiration, and grew normally with almost no malformation. The chimeric mice that had grown herein were characterized in that all cells, including germ line cells, were derived from the ES cells, and in that such mice can reproduce with a target animal and proliferate.

### [EXAMPLES]

The present invention will be hereafter described in detail by referring to examples. However, the examples are intended to merely illustrate the invention, and the invention is not limited by these examples.

### Example 1

### Production of inbred mice having substituted mitochondrial DNAs

### (1) Production of inbred strains B6-mtMus and B6-GFP-mtMus having Mus musculus musculus-type mitochondrial DNAs substituted by the back-crossing method

Female mice (8-week-old) were obtained by crossing 8-week-old male C57BL/6J inbred mice with 8-week-old female Mus musculus musculus wild-type mice. Back-crossing between the obtained female mice, again, 8-week-old male C57BL/6J inbred mice was repeatedly carried out. By repeating crossing 12 times or more, B6-mtMus inbred mice were produced wherein mitochondrial DNAs have been substituted with those of Mus musculus musculus type. In addition, the mitochondrial DNAs were analyzed by the Nested-PCR method (Kaneda H et al., PNAS 92, 4542-4546 (1995)), so that it was confirmed that the DNAs had been substituted with those of Mus musculus musculus type.

Furthermore, female B6-mtMus (N18) mice were crossed with male B6-GFP#4 mice (Ichida et al.), thereby producing B6-GFP-mtMus mice.

### (2) Production of inbred disease model mice C57BL/10J-mdx-mtMus (mdx-mtMus) by pronuclei replacement method

A 3-week-old female mdx inbred disease model mouse (C57BL/10-mdx) and a 3-week-old female hybrid mice (hybrid mice (CBF1-mtMus) resulting from ♀BALB-mtMus×♂C57BL/6) having Mus musculus musculus-type mitochondrial DNAs of a 3-week-old wild-type mouse were subjected to superovulation induction treatment (pregnant mare serum gonadotropin (PMSG) was administered at the rate of 5 IU/0.05 ml/mouse, and 48 hours later human chorionic gonadotropin (hCG) was administered at the rate of 5 IU/0.05 ml/mouse). The female hybrid mice were allowed to live together with 8-week-old male ICR mice for natural mating. At 24 hours after hCG administration, eggs were collected so as to prepare pronuclear-stage fertilized eggs. Using the thus obtained pronuclear-stage fertilized eggs, pronuclear replacement was conducted according to a standard method (McGrath J & Solter D, J. Exp. Zool. 228, 355-362 (1983)). Specifically, male and female pronuclei of the pronuclear-stage fertilized eggs, the donor fertilized eggs derived from the above C57BL/10-mdx inbred disease model mice, were introduced into enucleated pronuclear-stage fertilized eggs, the recipient fertilized eggs derived from the above CBF1-mtMus hybrid mice having the Mus musculus musculus-type mitochondrial DNAs of the wild-type mouse. After being subjected to nuclear fusion by electric pulse, the eggs were cultured overnight in an M16 medium supplemented with 0.1 mM EDTA (94.59 mM NaCl, 4.78 mM KCl, 1.71 mM CaCl₂, 1.19 mM KH₂PO₄, 1.19 mM MgSO₄, 25.07 mM NaHCO₃, 23.28 mM sodium lactate, 0.33 mM sodium pyruvate, 1 g/L glucose, 4 g/L BSA, 100 IU/mL penicillin, and 50 µg/mL streptomycin) under conditions of 37°C and 5% CO₂-5% O₂-90% N₂, and then transplanted in mouse oviducts. A total of 16 eggs were transplanted (8 eggs per oviduct) into the oviducts of female ICR mice on day 0.5 of pseudo-pregnancy (The female mice were crossed with vasoligated male mice, on the next day, plug confirmation was conducted, and the noon of this day was determined to be day 0.5). As a result, 3 female and 2 male pups were obtained. The 3 thus obtained female mice (8-week-old or older) as founders were allowed to live together with male mdx (C57BL/10-mdx) mice, thereby obtaining 2nd generation mice. Similarly, 2nd generation female mice were crossed with male mdx mice, thereby obtaining 3rd generation mice. The thus obtained 3rd generation mitochondrial DNAs were verified by the PCR-RFLP method. Common primers were designed within a D-loop (primer production referring to Kaneda H et al., PNAS 92, 4542-4546 (1995): 5'-AATTATTTTCCCCAAGC-3' (SEQ ID NO: 1 in Sequence Listing) and 5'-AGAAGAGGGGCATTG-3' (SEQ ID NO: 2 in Sequence Listing)). A 262-bp fragment obtained by amplification was treated with Dra1 restriction enzyme, and then unfragmented Mus musculus domesticus type DNAs and Mus musculus musculus type DNAs fragmented to a 122-bp fragment and a 140-bp fragment were verified. It was confirmed that the mitochondrial DNAs of the thus obtained 3rd generation mice had been substituted with those of Mus musculus musculus type, thereby producing inbred disease model mice C57BL/10J-mdx-mtMus (mdx-mtMus).

### Example 2

### Production of ES cells from inbred mice having substituted Mus musculus musculus-type mitochondrial DNAs

The inbred female B6-mtMus (N15) mice having the substituted Mus musculus musculus-type mitochondrial DNAs obtained in Example 1 (1) were naturally crossed with male C57BL/6 mice. The uterus of each mouse individual was perfused with an M2 medium on day 3 after plug confirmation, and then 20 blastocyst-stage embryos were collected. Using the obtained embryos, ES cells were established according to a standard method (Evans MJ and Kaufman MK, Nature 292, 154-156 (1981)). Specifically, the previously obtained embryos were transferred one by one onto feeder cells in a 4-well plate to which an ES medium (80%(v/v) DMEM, 20% (w/v) FCS, 1% (v/v) 100 mM pyruvate solution (Gibco Cat. 11360-070), 1% (v/v) 100X nonessential amino acid solution (Gibco Cat. 11140-027), 1 mM 2-mercaptoethanol (Sigma, Cat. No. M-6250), and 10³ U/mL LIF had been apportioned. The embryos were then cultured under conditions of 37°C and 5% CO₂-5% O₂-90% N₂. On day 5 of culture, ICM cell aggregation that had grown was selected. 0.125% trypsin/0.1 mM EDTA treatment was conducted to disperse the cells, and then the cells were seeded and cultured on feeder cells newly prepared in a 4-well plate. At the 2nd passage, only ES-cell-like colonies were selected, and then seeded and cultured on a new 4-well plate by the method same as that employed in the 1st time. At the 3rd passage, entire wells were treated with trypsin/EDTA, and then the cells were seeded on a new 3.5 cm culture plate, thereby obtaining 2 lines that were able to grow well. The cells that could reach this stage could then also stably grow, so that the cells were cryopreserved as a cell line.

Furthermore, 4 lines of ES cells were established by conducting similar manipulations using 6 blastocyst-stage embryos obtained from the inbred disease model mice mdx-mtMus obtained in Example 1 (2) and having the substituted Mus musculus musculus-type mitochondrial DNAs.

When the mitochondrial DNAs of the ES cells established from the above 2 mouse strains were tested, they were confirmed to be of Mus musculus musculus type.

### Example 3

### Preparation of tetraploid blastocyst-stage embryo

ICR mice subjected to superovulation induction treatment were naturally crossed with male mice of the same strain. Late 2-cell stage embryos were collected by the tubal perfusion method from mouse individuals for which plugs had been confirmed at 44 to 46 hours after the administration of human chorionic gonadotropin (hCG). The thus obtained late 2-cell stage embryos were subjected to fusion treatment by direct current electric pulse in 0.3 M mannitol (*Goku* manufactured by FUJIHIRA, and pulse conditions of 18 V, at intervals of 50 µsec and 999 µsec (3 times each)), thereby preparing tetraploid embryos. The tetraploid embryos were selected, and then cultured in a M16 medium at 37°C in 5% CO₂-Air for two days, thereby obtaining tetraploid blastocyst-stage embryos.

### Example 4

### Production of chimeric mice by tetraploid rescue method (1)

ES cells (gender: male) prepared from the prescribed B6-mtMus inbred mice were injected using a piezomicromanipulator into the tetraploid blastocyst-stage embryos obtained in Example 3. Approximately 10 to 15 ES cells were injected into a tetraploid blastocyst-stage embryo. 76 tetraploid blastocyst-stage embryos were subjected to injection. 71 tetraploid blastocyst-stage embryos into which the ES cells had been successfully injected were implanted into the uteri of 4 recipient ICR mice on day 2.5 after plug confirmation. On the night before delivery (night of day 18.5 of pregnancy), chimeric mouse fetuses were taken out by Cesarean section, and then resuscitated. After active movement of the fetuses was confirmed, they were raised by previously prepared foster mothers. The 13 thus obtained male newborns showed no malformation and conducted spontaneous respiration, and 4 of these mice grew normally.

### Example 5

### Production of chimeric mice by tetraploid rescue method (2)

ES cells (gender: male) prepared from the prescribed mdx-mtMus inbred disease model mice were injected using a piezomicromanipulator into the tetraploid blastocyst-stage embryos obtained in Example 3. Approximately 10 to 15 ES cells were injected into each tetraploid blastocyst-stage embryo. 108 tetraploid blastocyst-stage embryos were subjected to injection. 89 tetraploid blastocyst-stage embryos into which the ES cells had been successfully injected were transplanted into the uteri of 5 recipient ICR mice on day 2.5 after plug confirmation. On the night before delivery (night of day 18.5 of pregnancy), fetuses were taken out by Cesarean section, and then resuscitated. After active movement of the fetuses was confirmed, they were raised by previously prepared foster parents. The 19 thus obtained male newborns showed almost no malformation and conducted spontaneous respiration, and 11 of these mice grew normally.

### Example 6

### Production of chimeric mice by tetraploid rescue method (3)

ES cells (gender: female) prepared from the prescribed B6-GFP-mtMus inbred mice were injected using a piezomicromanipulator into the tetraploid blastocyst-stage embryos obtained in Example 3. Approximately 10 to 15 ES cells were injected into each tetraploid blastocyst-stage embryo. 75 tetraploid blastocyst-stage embryos were subjected to injection. 75 tetraploid blastocyst-stage embryos to which the ES cells had been successfully injected were implanted in the uteri of 3 recipient ICR mice on day 2.5 after plug confirmation. On the night before delivery (night of day 18.5 of pregnancy), chimeric mouse fetuses were taken out by Cesarean section, and then resuscitated. After active movement of the fetuses was confirmed, they were raised by previously prepared foster parents. 8 out of 12 female newborns showed no malformation and conducted spontaneous respiration. 7 mice died because of child neglect or were killed by recipient mice. The remaining 1 mouse pups grew normally.

### Example 7

### Confirmation of proliferation of chimeric mice produced by tetraploid rescue method

Two male chimeric mice obtained from ES cells prepared from the inbred disease model mdx-mtMus obtained in Example 1 were crossed with 2 normal female mdx-mice, thereby obtaining 38 newborns. These mice showed no malformation and grew smoothly. Furthermore, each of all the mouse individuals showed typical features of mdx (C57BL/10-mdx) inbred disease model mice. Thus, it could be confirmed that the chimeric mice obtained by the method of the present invention can leave normal pups in future generations.

### [Industrial Applicability]

Implementation of the present invention enables reproducible chimeric animals, particularly inbred chimeric animals, and further particularly inbred chimeric mouse individuals to be efficiently obtained. In particular, the implementation of the present invention enables to efficiently produce inbred animals while avoiding death because of respiratory disturbances immediately after the birth of inbred animals and particularly chimeric mice derived from totipotent cells such as ES cells or nuclear transferred embryos derived from inbred animals used in producing chimeric animals obtained by the tetraploid rescue method. The thus obtained chimeric mouse is a reproducible inbred mouse which has mitochondrial DNAs adapted to nuclear DNAs and wherein totipotent cells are transmitted to a germ line.

## Claims

1. A method for producing an animal derived from manipulated embryo derived from totipotent cells, comprising using cells having adapted type mitochondrial DNAs introduced therein which adapt to nuclear DNAs.

2. The method for producing an animal of claim 1, wherein the animal derived from the manipulated embryo derived from the totipotent cells is a chimeric animal.

3. The method for producing an animal of claim 2, wherein the chimeric animal is an inbred chimeric animal.

4. The method for producing a chimeric animal of any one of claims 1 to 3, comprising using totipotent cells having adapted type mitochondrial DNAs introduced therein and an untreated host embryo, an untreated totipotent cells and a host embryo having adapted type mitochondrial DNAs introduced therein, or totipotent cells having adapted type mitochondrial DNAs introduced therein and a host embryo having adapted type mitochondrial DNAs introduced therein.

5. The method for producing an animal of claim 4, comprising using a tetraploid fertilized egg as a host embryo for an animal and totipotent cells as cells having adapted type mitochondrial DNAs introduced therein.

6. The method for producing an animal of claim 5 according to a tetraploid rescue method, comprising injecting the totipotent cells having the adapted type mitochondrial DNAs introduced therein into the blastocyst of a tetraploid fertilized egg.

7. The method for producing a chimeric animal of any one of claims 1 to 6, wherein the cells having the adapted type mitochondrial DNAs introduced therein is cells which have the adapted type mitochondrial DNAs as a result of substitution.

8. A method for producing a clone individual by nuclear transfer using a recipient oocyte having adapted type mitochondrial DNAs that adapt to a nuclear DNAs of a donor cell.

9. The method for producing an animal of any one of claims 1 to 8, wherein nuclear-mitochondrial incompatibility results in respiratory disturbances immediately after birth.

10. The method for producing an animal of any one of claims 1 to 9, wherein the adapted type mitochondrial DNAs are mitochondrial DNAs derived from a wild type.

11. The production method of any one of claims 1 to 10, wherein the animal is a mouse.

12. The method for producing an animal of any one of claims 1 to 11, wherein the method for substituting mitochondrial DNAs with desired mitochondrial DNAs is either a back-crossing method or a pronuclei replacement method.

13. The method for producing an animal of any one of claims 9 to 12, wherein the desired mitochondrial DNAs are of a wild-type mouse Mus musculus musculus type.

14. A reproducible chimeric animal, which is produced by any one of the methods of claims 1 to 13, and wherein mitochondrial DNAs are introduced into or substituted with adapted type DNAs, and totipotent cells can be transmitted to a germ line.

15. The animal of claim 14, wherein the animal is a mouse.

16. A totipotent cell for producing a reproducible genetically manipulated animal, wherein mitochondrial DNAs are of adapted type, and its character is transmitted to a germ line.

17. The totipotent cell of claim 16, wherein adaptation with nuclear DNAs causes a decrease in respiratory disturbances immediately after birth.

18. The totipotent cell of claim 16 or 17, wherein adapted type mitochondrial DNAs have been introduced as mitochondrial DNAs.

19. The totipotent cell of claim 18, wherein the adapted type mitochondrial DNAs areof a wild-type mouse Mus musculus musculus type.

20. The embryonic stem cell of any one of claims 16 to 19, wherein the genetically manipulated animal is a mouse.
